(19) Europäisches Patentamt
European Patent Office
Office européen des brevets

(11) **EP 4 693 331 A1**

(12) **EUROPEAN PATENT APPLICATION**
published in accordance with Art. 153(4) EPC

(43) Date of publication:
**11.02.2026  Bulletin 2026/07**

(21) Application number: **24779511.5**

(22) Date of filing: **14.03.2024**

(51) International Patent Classification (IPC):
**G16H 50/00** (2018.01)    **G06N 20/00** (2019.01)

(52) Cooperative Patent Classification (CPC):
**G06N 20/00; G16H 50/00**

(86) International application number:
**PCT/JP2024/009956**

(87) International publication number:
**WO 2024/203382 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **27.03.2023   JP 2023050360**

(71) Applicant: **NEC Solution Innovators, Ltd.
Koto-ku, Tokyo 136-8627 (JP)**

(72) Inventors:
• **NOMURA, Hitoshi
Tokyo 136-8627 (JP)**
• **KATO, Shintaro
Tokyo 136-8627 (JP)**

(74) Representative: **Betten & Resch
Patent- und Rechtsanwälte PartGmbB
Maximiliansplatz 14
80333 München (DE)**

(54) **LEARNING MODEL GENERATION DEVICE, INSPECTION VALUE PREDICTION DEVICE, LEARNING MODEL GENERATION METHOD, INSPECTION VALUE PREDICTION METHOD, AND COMPUTER-READABLE RECORDING MEDIUM**

(57)    This learning model generation device 10 is equipped with a learning model generation unit 11 which, when a function expressing a change in an inspection value obtained by inspecting a person is set, generates a learning model in which the inspection value is the explanatory variable and the parameter is the objective variable, by performing machine learning using inspection values of sample people and parameters of the function for the sample people as training data.

Fig.1

## Description

Technical Field

The present disclosure relates to an inspection value prediction apparatus and an inspection value prediction method for predicting a future inspection value of a user. The present disclosure relates to a learning model generation apparatus and a learning model generation method for generating a learning model used for prediction of an inspection value. Furthermore, the present disclosure relates to a computer-readable recording medium in which a program for implementing these apparatuses and methods is recorded.

Background Art

In recent years, utilization of big data in the medical and healthcare areas has become active for lifestyle measures. The big data analysis can be expected to predict the future health condition of each employee, recommend specific improvement measures, and the like.

For example, NPL 1 discloses a system that predicts a value of an inspection item of a user using a learning model obtained by machine learning a relationship between an inspection value and a lifestyle. In the system disclosed in NPL 1, when data indicating the user's lifestyle is input to the learning model, the learning model outputs a future inspection value of the user. The user can know how the inspection value changes in the future when continuing the current lifestyle.

Citation List

Non Patent Literature

NPL 1: Atsushi Taniguchi, Hiroshi Tamano, "Learning Medical Checkup Prediction
Model with Interpretable Interannual Prediction", FIT2018 (17th Information Science and Technology Forum), 2018, second issue, p. 45-50

Summary of Invention

Technical Problem

When predicting the inspection value for three years, the system disclosed in NPL 1 described above first predicts the inspection value for one year from the present, and then predicts one year to two years using the predicted inspection value for one year. Furthermore, the system disclosed in NPL 1 described above predicts two to three years later using the predicted two-year inspection value.

For this reason, in the system disclosed in NPL 1 described above, the tendency of change may be different in each of the initially predicted inspection value after one year from the present, the inspection value after one year to two years, and the inspection value after two years to three years. As a result, interpretability in the learning model decreases. For example, it is assumed that the inspection value increases from the present to one year later, decreases from one year to two years later, and further increases from two years to three years later. In this case, interpretability in the learning model decreases, and it becomes difficult to interpret behavior of the learning model.

An object of the present disclosure is to improve interpretability by indicating a certain tendency in prediction of an inspection value of a user.

Solution to Problem

In order to achieve the above object, a learning model generation apparatus according to one aspect of the present disclosure includes a learning model generation unit for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

In order to achieve the above object, an inspection value prediction apparatus according to one aspect of the present disclosure includes:

an inspection value acquisition unit for acquiring an inspection value of a person to be predicted;
a parameter prediction unit for inputting the acquired inspection value to a learning model in which a relationship between an inspection value and a parameter of a function representing a transition of the inspection value is machine-learned and predicting the parameter of the person to be predicted; and
an inspection value prediction unit for inputting the predicted parameter to the function and predicts a future inspection value of the person to be predicted.

In order to achieve the above object, a learning model generation method according to one aspect of the present disclosure includes a learning model generation step for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

In order to achieve the above object, an inspection value prediction method according to one aspect of the present disclosure includes:

an inspection value acquisition step for acquiring an inspection value of a person to be predicted;

a parameter prediction step for inputting the acquired inspection value to a learning model in which a relationship between an inspection value and a parameter of a function representing a transition of the inspection value is machine-learned and predicting the parameter of the person to be predicted; and

an inspection value prediction step for inputting the predicted parameter to the function and predicts a future inspection value of the person to be predicted.

Furthermore, in order to achieve the above object, a first computer-readable recording medium according to one aspect of the present disclosure has recorded therein a program including an instruction for causing a computer to execute, in a case where a function representing a transition of an inspection value obtained from inspection on a person is set, a learning model generation step for executing machine learning with, as training data, an inspection value of a sample person and a parameter of the function for the sample person and generating a learning model with the inspection value as an explanatory variable and the parameter as an objective variable.

Furthermore, in order to achieve the above object, a second computer-readable recording medium according to one aspect of the present disclosure has recorded therein a program including an instruction for causing a computer to execute:

an inspection value acquisition step for acquiring an inspection value of a person to be predicted;

a parameter prediction step for inputting the acquired inspection value to a learning model in which a relationship between an inspection value and a parameter of a function representing a transition of the inspection value is machine-learned and predicting the parameter of the person to be predicted; and

an inspection value prediction step for inputting the predicted parameter to the function and predicts a future inspection value of the person to be predicted.

Advantageous Effects of Invention

As described above, according to the present disclosure, it is possible to improve interpretability by indicating a certain tendency in prediction of an inspection value of a user.

Brief Description of Drawings

[Fig. 1] Fig. 1 is a configuration diagram illustrating a schematic configuration of an example of a learning model generation apparatus.

[Fig. 2] Fig. 2 is a configuration diagram specifically illustrating a configuration of an example of the learning model generation apparatus.

[Fig. 3] Fig. 3 is a diagram illustrating an example of a function.

[Fig. 4] Fig. 4 is a diagram illustrating an example of training data.

[Fig. 5] Fig. 5 is a flowchart illustrating an operation of an example of the learning model generation apparatus.

[Fig. 6] Fig. 6 is a configuration diagram illustrating a configuration of a modification of the learning model generation apparatus.

[Fig. 7] Fig. 7 is a configuration diagram illustrating a schematic configuration of an example of an inspection value prediction apparatus.

[Fig. 8] Fig. 8 is a configuration diagram specifically illustrating a configuration of an example of the inspection value prediction apparatus.

[Fig. 9] Fig. 9 is a flowchart illustrating an operation of an example of the inspection value prediction apparatus.

[Fig. 10] Fig. 10 is a block diagram illustrating an example of a computer that implements the learning model generation apparatus and the inspection value prediction apparatus.

Example Embodiment

(First example embodiment)

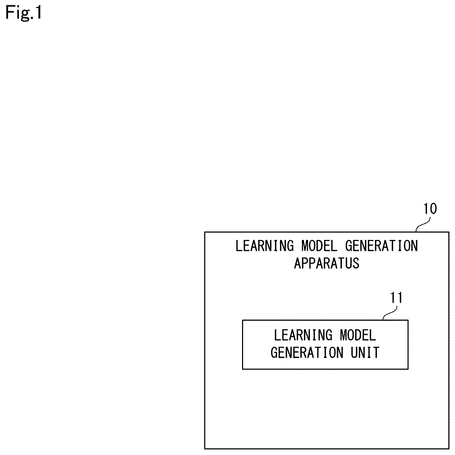

Hereinafter, in a first example embodiment, a learning model generation apparatus, a learning model generation method, and a program will be described with reference to Figs. 1 to 5.

[Apparatus configuration]

First, a schematic configuration of a learning model generation apparatus will be described with reference to Fig. 1. Fig. 1 is a configuration diagram illustrating a schematic configuration of an example of the learning model generation apparatus.

A learning model generation apparatus 10 according to the first example embodiment illustrated in Fig. 1 is an apparatus that generates a learning model for predicting a future inspection value of a user. As illustrated in Fig. 1, the learning model generation apparatus 10 includes a learning model generation unit 11.

First, a function representing a transition of an inspection value obtained from an inspection on a person is set as a function. In this case, the learning model generation unit 11 executes machine learning using, as training data, the inspection value of the sample person and the parameter (hereinafter, referred to as a "function parameter") of the function for the sample person to generate a learning model. The learning model uses the inspection value as an explanatory variable and the parameter of the function as an objective variable.

As described above, in the first example embodiment, the function representing the transition of the inspection value is set, and the learning model in which the relationship between the inspection value and the function parameter is machine-learned is generated. Therefore, first, a function parameter is predicted by the learning model, and a future inspection value is predicted from the predicted function parameter. Therefore, according to the first example embodiment, since a certain tendency is shown in the prediction of the inspection value of the user, interpretability can be improved.

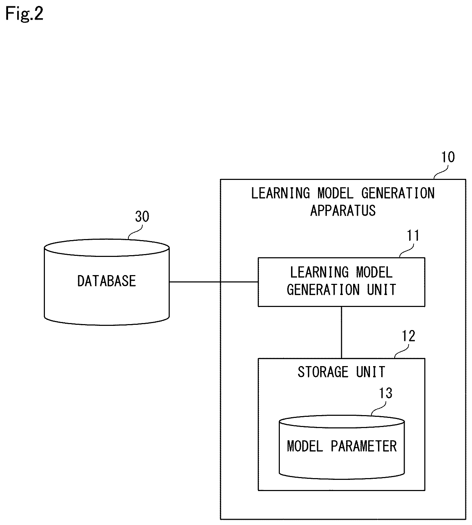

Next, a configuration and a function of the learning model generation apparatus 10 will be specifically described with reference to Figs. 2 to 4. Fig. 2 is a configuration diagram specifically illustrating a configuration of an example of the learning model generation apparatus.

As illustrated in Fig. 2, in the first example embodiment, the learning model generation apparatus 10 is data-communicably connected to a database 30 via a network or the like. The database 30 stores training data for machine learning.

Furthermore, as illustrated in Fig. 2, the learning model generation apparatus 10 includes a storage unit 12 in addition to the learning model generation unit 11. The storage unit 12 stores parameters (hereinafter, referred to as a "model parameter") 13 for constructing the learning model.

The "generation of a learning model" in the present invention means generation or update of a model parameter of a learning model. That is, the learning model generation apparatus 10 generates or updates the model parameters 13 of the learning model by executing machine learning, and stores the generated or updated model parameters 13 in the storage unit 12. As a result, a learning model is generated. The learning model is generated for each inspection item, that is, for each inspection value.

In the first example embodiment, a function representing the transition of the inspection value after the set period elapses is used as the function. Specific examples of the function include a function expressed by the following Expression 1.

$$(\text{Expression 1})$$

$$y = a \log t + b$$

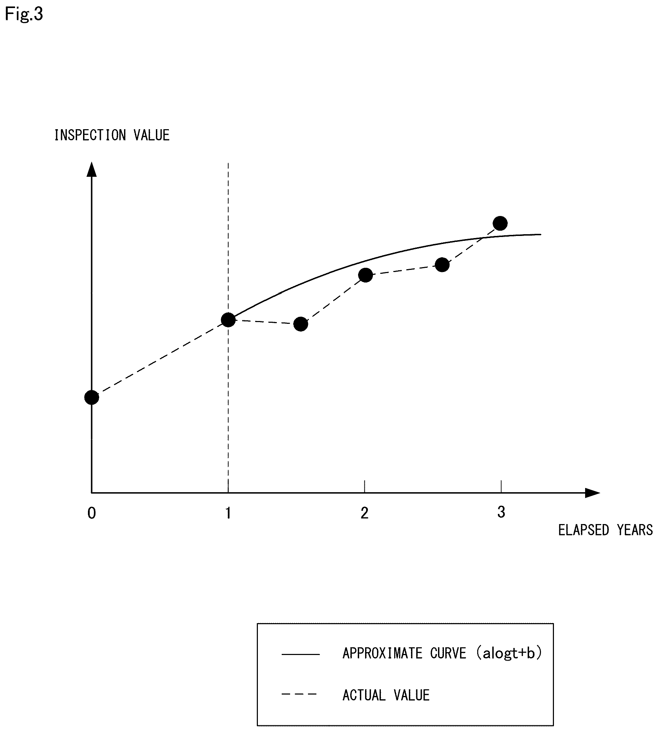

A function used in the first example embodiment will be described with reference to Fig. 3. Fig. 3 is a diagram illustrating an example of a function.

The graph illustrated in Fig. 3 is a graph in which the vertical axis represents the inspection value and the horizontal axis represents the elapsed years. In the example of Fig. 3, the above-described set period is one year. The reference time "0" is arbitrarily set. The function shown in Expression 1 above is set based on the set period of one year, and the vertical axis passing through one year is the baseline. In Fig. 3, black circles indicate actual values. The function shown in Expression 1 above is set as an approximate curve of actual values after two years.

In Expression 1 above, b is relevant to the actual value on the baseline. Then, a is a function parameter determined by an inspection value after two years. In other words, the function parameter a is a parameter determined by the transition of the past inspection value of the person over time, and has a different value for each person.

Therefore, in the first example embodiment, machine learning is performed using the function parameter a of the above Expression 1 as an objective variable. Furthermore, in the first example embodiment, the content of the questionnaire is also used as the explanatory variable in addition to the above-described inspection value. Specific examples of the contents of the questionnaire include the lifestyle of a person, for example, the presence or absence of current or past smoking, the amount of alcohol consumed per day, the frequency of exercise (frequency of exercise per unit time), and the like.

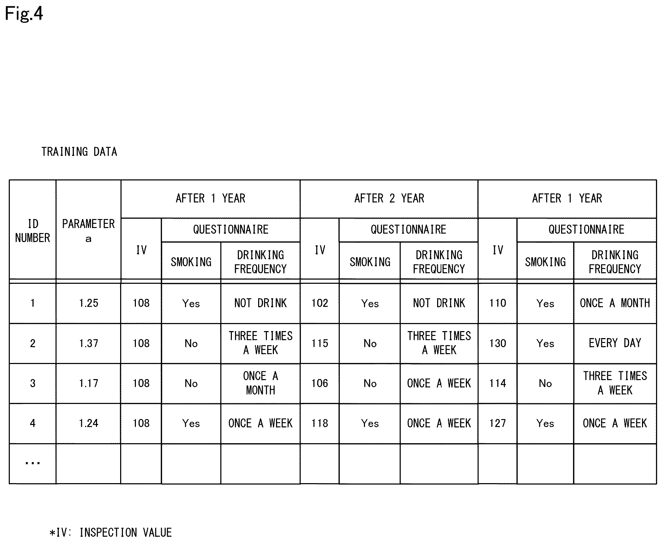

For example, as illustrated in Fig. 4, the database 30 stores, as training data, set data of the function parameter a, past data of the inspection value after one year from the reference time, and the content of the questionnaire at the time when the inspection value is acquired for each sample person. Fig. 4 is a diagram illustrating an example of training data.

In the first example embodiment, the learning model generation unit 11 executes machine learning using, as training data, the contents of a questionnaire of a sample person in addition to the inspection value and the function parameter a. Therefore, the learning model generation unit 11 reads the set data described above as training data from the database 30, and executes machine learning using the read set data.

Specifically, the learning model generation unit 11 constructs a learning model such as a neural network on the memory using the machine learning engine, and inputs the inspection value and the content of the questionnaire to the constructed learning model.

Then, the learning model generation unit 11 calculates a difference between the output value of the learning model and the function parameter a serving as the teacher data, and updates the model parameter 13 in the learning model so that the calculated difference approaches 0. Thereafter, when the update of the model parameters 13 with all the training data is completed, the learning model generation unit 11 stores the final model parameters 13 in the storage unit 12.

[Apparatus operation]

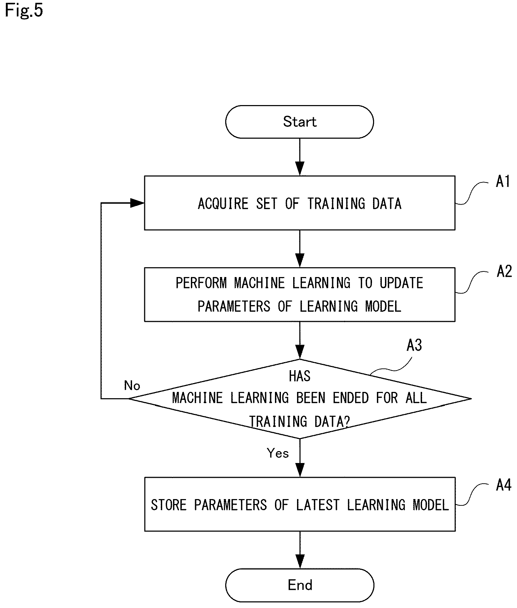

Next, the operation of the learning model generation apparatus 10 in the first example embodiment will be described with reference to Fig. 5. Fig. 5 is a flowchart illustrating an operation of an example of the learning model generation apparatus. In the following description, Figs. 1 to 4 will be appropriately referred to. In the first example embodiment, the learning model generation method is performed by operating the learning model generation apparatus 10. Therefore, the description of the learning model generation method in the first example embodiment is replaced with the following description of the operation of the learning model generation apparatus 10.

First, as a premise, the function shown in Expression 1 above is set. Furthermore, the database 30 stores, as training data, a function parameter a, past data of the inspection value after one year from the reference time, and the content of the questionnaire at the time when the inspection value is acquired for each sample person.

As illustrated in Fig. 5, first, the learning model generation unit 11 acquires a set of training data from the database 30 (step A1).

Next, the learning model generation unit 11 executes machine learning using the training data acquired in step A1 and updates the parameters of the learning model (step A2).

Specifically, in step A2, the learning model generation unit 11 constructs a learning model such as a neural network on a memory using a machine learning engine. Then, the learning model generation unit 11 inputs the inspection value included in the training data and the contents of the questionnaire to the constructed learning model.

Furthermore, the learning model generation unit 11 calculates a difference between the output value of the learning model and the function parameter a serving as teacher data. Subsequently, the learning model generation unit 11 updates the model parameter 13 of the learning model so that the calculated difference approaches 0.

Next, the learning model generation unit 11 determines whether machine learning has been completed for all the training data (step A3).

As a result of the determination in step A3, in a case where the machine learning has not been completed for all sets of training data, the learning model generation unit 11 executes step A1 again to acquire one set of new training data.

On the other hand, as a result of the determination in step A3, in a case where the machine learning has been completed for all the sets of training data, the learning model generation unit 11 stores the model parameters 13 of the final learning model that has been updated with all the training data in the storage unit 12 (step A4).

As described above, in the first example embodiment, the function representing the transition is set for the inspection value after one year, which is likely to deteriorate interpretability in the related art, and the learning model in which the relationship between the inspection value and the function parameter is machine-learned is generated. In a case where a future inspection value is predicted using this learning model, as described in a second example embodiment to be described later, a function parameter is predicted by the learning model, and a future inspection value is predicted from the predicted function parameter, so that a prediction result with high interpretability can be obtained. That is, according to the first example embodiment, a certain tendency can be indicated in the prediction of the inspection value of the user, and the interpretability can be improved.

[Program]

The program in the first example embodiment may be a program that causes a computer to execute steps A1 to A4 illustrated in Fig. 5. By installing and executing this program in the computer, the learning model generation apparatus 10 and the learning model generation method according to the first example embodiment can be implemented. In this case, the processor of the computer functions as the learning model generation unit 11 and performs processing.

In the first example embodiment, the storage unit 12 may be achieved by storing data files constituting the storage unit in a storage device such as a hard disk provided in a computer, or may be achieved by a storage device of another computer.

Examples of the computer include a smartphone and a tablet terminal device in addition to a general-purpose PC. The program according to the first example embodiment may be executed by a computer system constructed by a plurality of computers.

[Modification]

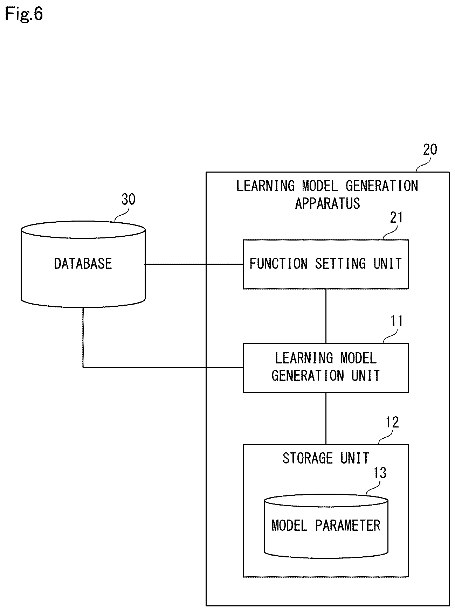

Next, a modification of the first example embodiment will be described with reference to Fig. 6. Fig. 6 is a configuration diagram illustrating the configuration of the modification of the learning model generation apparatus.

Similarly to the learning model generation apparatus 10, a learning model generation apparatus 20 in the modification illustrated in Fig. 6 is an apparatus that generates a learning model for predicting a future inspection value of the user.

As illustrated in Fig. 6, similarly to the learning model generation apparatus 10, the learning model generation apparatus 20 also includes a learning model generation unit 11 and a storage unit 12. However, in the modification, the learning model generation apparatus 20 further includes a function setting unit 21 in addition to these.

The function setting unit 21 sets a function based on the transitions of the inspection values of a plurality of persons for a specific period. Specifically, also in the modification, the function is a function representing the transition of the inspection value after the set period elapses, and the set period is one year. In this case, the inspection value for the specific period is an inspection value for several years after one year, which is the set period.

First, the function setting unit 21 acquires inspection values for a specific period of a plurality of persons from the database 30. Subsequently, the function setting unit 21 plots the acquired transition of the inspection value of each person in a graph in which the vertical axis represents the inspection value and the horizontal axis represents the elapsed years. Then, the function setting unit 21 specifies an approximate function from the plotted inspection value using, for example, the least squares method or the like, and sets the specified function.

That is, in the examples of Figs. 1 and 2 described above, the function is set in advance, but in the modification, the function setting unit 21 can set the function from the inspection value of each person as a sample. Therefore, according to the modification, a learning model with higher prediction accuracy can be generated.

(Second example embodiment)

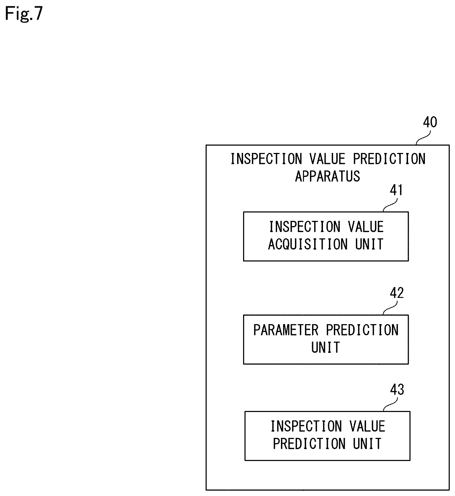

Next, an inspection value prediction apparatus, an inspection value prediction method, and a program in a second example embodiment will be described with reference to Figs. 7 to 9.

[Apparatus configuration]

First, a schematic configuration of an inspection value prediction apparatus will be described with reference to Fig. 7. Fig. 7 is a configuration diagram illustrating the schematic configuration of the example of the inspection value prediction apparatus.

An inspection value prediction apparatus 40 according to the second example embodiment illustrated in Fig. 7 is an apparatus for predicting a future inspection value of a user. As illustrated in Fig. 6, the inspection value prediction apparatus 40 includes an inspection value acquisition unit 41, a parameter prediction unit 42, and an inspection value prediction unit 43.

The inspection value acquisition unit 41 acquires an inspection value of a person to be predicted. The parameter prediction unit 42 inputs the acquired inspection value to the learning model and predicts a parameter (function parameter) of a person to be predicted. The learning model performs machine learning on the relationship between the inspection value and the function parameter of the function representing the transition of the inspection value. The inspection value prediction unit 43 inputs the predicted function parameter to the function and predicts a future inspection value of the person to be predicted.

As described above, in the second example embodiment, first, the parameter of the function representing the transition of the inspection value is predicted by the learning model, and then, the future inspection value is predicted using the function to which the parameter is input. Therefore, according to the second example embodiment, since a certain tendency is shown in the prediction of the inspection value of the user, interpretability can be improved.

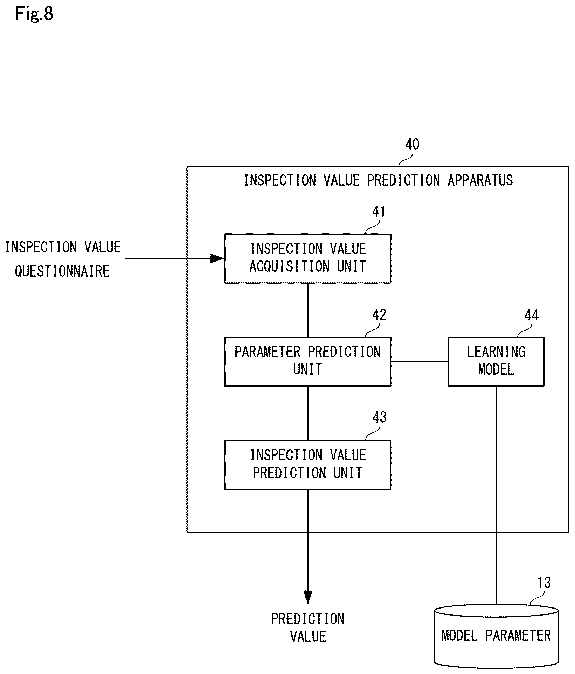

Next, a configuration and a function of an example of the inspection value prediction apparatus 40 will be specifically described with reference to Fig. 8. Fig. 8 is a configuration diagram specifically illustrating a configuration of an example of the inspection value prediction apparatus.

As illustrated in Fig. 8, in the second example embodiment, the inspection value prediction apparatus 40 includes a learning model 44 in addition to the inspection value acquisition unit 41, the parameter prediction unit 42, and the inspection value prediction unit 43 described above.

Also in the second example embodiment, as the function, a function representing the transition of the inspection value after the set period elapses, specifically, a function expressed by the above Expression 1 is used, and as the function parameter, the function parameter a expressed by Expression 1 is used. In a case where a function other than the function expressed by the above Expression 1 is used as the function in the first example embodiment, a function other than the function expressed by the above Expression 1 is also used in the second example embodiment.

The learning model 44 is a learning model generated in the first example embodiment. In the learning model 44, the function parameter of the function is set as an objective variable, and the inspection value and the contents of the questionnaire are set as explanatory variables. Specifically, the learning model 44 is a neural network or the like constructed on the memory by the machine learning engine using the model parameters 13 updated in the first example embodiment.

In the second example embodiment, the inspection value acquisition unit 41 acquires a questionnaire of a person to be predicted in addition to the inspection value of the person to be predicted described above. Then, in the second example embodiment, the parameter prediction unit 42 inputs the acquired inspection value and the content of the questionnaire to the learning model 44 and predicts the function parameter a of the person to be predicted.

In the second example embodiment, the inspection value prediction unit 43 inputs the predicted function parameter a to the function expressed by the above Expression 1. Then, the inspection value prediction unit 43 inputs the value t of the period to the function to which the function parameter a has been input, and predicts the future inspection value of the person to be predicted.

Thereafter, the inspection value prediction unit 43 presents the predicted inspection value on a screen of a display device directly connected to the inspection value prediction apparatus 40, a screen of a terminal device connected to the inspection value prediction apparatus 40 via a network, and the like.

[Apparatus operation]

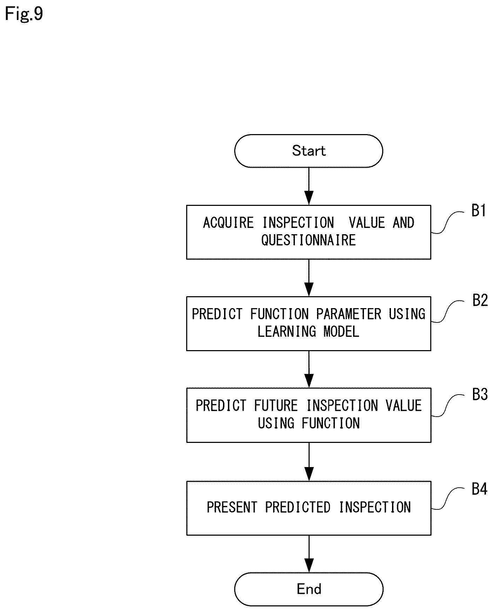

Next, the operation of the inspection value prediction apparatus 40 will be described with reference to Fig. 9. Fig. 9 is a flowchart illustrating an operation of an example of the inspection value prediction apparatus. Figs. 7 and 8 will be appropriately referred to in the following description. In the second example embodiment, the inspection value prediction method is performed by operating the inspection value prediction apparatus 40. Therefore, the description of the inspection value prediction method in the second example embodiment is replaced with the following description of the operation of the inspection value prediction apparatus 40.

As illustrated in Fig. 9, first, the inspection value acquisition unit 41 acquires an inspection value and a questionnaire of a person to be predicted (step B1).

Next, the parameter prediction unit 42 inputs the inspection value acquired in step B1 and the contents of the questionnaire to the learning model 44, acquires the output of the learning model 44, and predicts the function parameter a of the person to be predicted (step B2). Specifically, when the inspection value acquired in step B1 and the content of the questionnaire are input, the learning model 44 outputs the predicted value of the function parameter a. The parameter prediction unit 42 performs prediction by acquiring the output prediction value.

Next, the inspection value prediction unit 43 inputs the predicted function parameter a to the function expressed by the above Expression 1 to predict the future inspection value of the person to be predicted (step B3). Specifically, the inspection value prediction unit 43 inputs the value t of the period to the function to which the function parameter a has been input, and predicts the future inspection value of the person to be predicted.

Thereafter, the inspection value prediction unit 43 presents the predicted inspection value on a screen of the display device, a screen of the terminal device, or the like (step B4).

As described above, in the second example embodiment, since the function parameter a is predicted by the learning model 44 and the future inspection value is predicted from the predicted function parameter a, it is possible to obtain a prediction result with high interpretability for the inspection value after one year, which is likely to deteriorate in interpretability in the related art. Therefore, according to the second example embodiment, a certain tendency can be indicated in the prediction of the inspection value of the user, and the interpretability can be improved.

[Program]

The program in the second example embodiment may be a program that causes a computer to execute steps B1 to B4 illustrated in Fig. 9. By installing and executing this program in a computer, the inspection value prediction apparatus 40 and the inspection value prediction method according to the second example embodiment can be achieved. In this case, the processor of the computer functions as the inspection value acquisition unit 41, the parameter prediction unit 42, and the inspection value prediction unit 43, and performs processing.

Examples of the computer include a smartphone and a tablet terminal device in addition to a general-purpose PC. The program according to the second example embodiment may be executed by a computer system constructed by a plurality of computers. In this case, for example, each computer may function as any of the inspection value acquisition unit 41, the parameter prediction unit 42, and the inspection value prediction unit 43.

[Physical configuration]

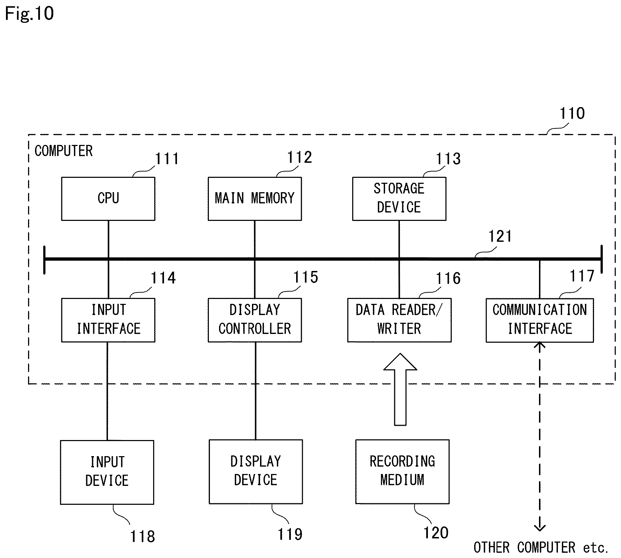

Here, a computer that implements the learning model generation apparatus 10 or the inspection value prediction apparatus 40 by executing a program in the first and second example embodiments will be described with reference to Fig. 10. Fig. 10 is a block diagram illustrating an example of a computer that implements the learning model generation apparatus and the inspection value prediction apparatus.

As illustrated in Fig. 10, a computer 110 includes a central processing unit (CPU) 111, a main memory 112, a storage device 113, an input interface 114, a display controller 115, a data reader/writer 116, and a communication interface 117. These units are data-communicably connected to each other via a bus 121.

The computer 110 may include a graphics processing unit (GPU) or a field-programmable gate array (FPGA) in addition to the CPU 111 or instead of the CPU 111. In this aspect, the GPU or the FPGA can execute the program in the example embodiment.

The CPU 111 develops the program according to the example embodiment, which is stored in the storage device 113 and configured by codes, in the main memory 112, and executes each code in a predetermined order to perform various operations. The main memory 112 is typically a volatile storage device such as a dynamic random access memory (DRAM).

The program in the example embodiment is provided in a state of being stored in a computer-readable recording medium 120. The program in the present example embodiment may be distributed on the Internet connected via the communication interface 117.

Specific examples of the storage device 113 include a semiconductor storage device such as a flash memory in addition to a hard disk drive. The input interface 114 mediates data transmission between the CPU 111 and an input device 118 such as a keyboard and a mouse. The display controller 115 is connected to a display device 119 and controls display on the display device 119.

The data reader/writer 116 mediates data transmission between the CPU 111 and the recording medium 120, and reads a program from the recording medium 120 and writes a processing result of the computer 110 into the recording medium 120. The communication interface 117 mediates data transmission between the CPU 111 and another computer.

Specific examples of the recording medium 120 include general-purpose semiconductor storage devices such as a Compact Flash (CF) (registered trademark) and a secure digital (SD), a magnetic recording medium such as a flexible disk, and an optical recording medium such as a compact disk read only memory (CD-ROM).

The learning model generation apparatus and the inspection value prediction apparatus can also be achieved by using hardware relevant to each unit, for example, an electronic circuit, instead of a computer in which a program is installed. Furthermore, a part of the learning model generation apparatus and the inspection value prediction apparatus may be achieved by a program, and the remaining part may be achieved by hardware. In the example embodiment, the computer is not limited to the computer illustrated in Fig. 10.

Some or all of the above-described example embodiments can be expressed by (Supplementary Note 1) to (Supplementary Note 30) described below, but are not limited to the following description.

(Supplementary Note 1)

A learning model generation apparatus including a learning model generation unit for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

(Supplementary Note 2)

The learning model generation apparatus according to Supplementary Note 1, in which the learning model generation unit further executes machine learning by using, as the training data, a content of a questionnaire of the sample person, and generates the learning model with the inspection value and the content of the questionnaire as an explanatory variable and the parameter as an objective variable.

(Supplementary Note 3)

The learning model generation apparatus according to Supplementary Note 2, in which the questionnaire includes a lifestyle of the sample person.

(Supplementary Note 4)

The learning model generation apparatus according to Supplementary Note 1, in which the function is a function representing a transition of the inspection value after a set period elapses.

(Supplementary Note 5)

The learning model generation apparatus according to Supplementary Note 1, further including a function setting unit for setting the function based on transitions of inspection values of a plurality of persons for a specific period.

(Supplementary Note 6)

The learning model generation apparatus according to Supplementary Note 1, in which a parameter of the function for the sample person is obtained from a transition of a past inspection value of the sample person over time.

(Supplementary Note 7)

An inspection value prediction apparatus including:

an inspection value acquisition unit for acquiring an inspection value of a person to be predicted;
a parameter prediction unit for inputting the acquired inspection value to a learning model in which a relationship between an inspection value and a parameter of a function representing a transition of the inspection value is machine-learned and predicting the parameter of the person to be predicted; and
an inspection value prediction unit for inputting the predicted parameter to the function and predicts a future inspection value of the person to be predicted.

(Supplementary Note 8)

The inspection value prediction apparatus according to Supplementary Note 7, in which

the learning model performs machine learning on a relationship with a content of a questionnaire of a person in addition to an inspection value and a parameter of a function representing a transition of the inspection value,
the inspection value acquisition unit further acquires a questionnaire of the person to be predicted, and

the parameter prediction unit inputs the acquired inspection value and the content of the questionnaire to the learning model to predict the parameter of the person to be predicted.

(Supplementary Note 9)

The inspection value prediction apparatus according to Supplementary Note 8, in which the questionnaire includes a lifestyle of a person.

(Supplementary Note 10)

The inspection value prediction apparatus according to Supplementary Note 7, in which the function is a function representing a transition of the inspection value after a set period elapses.

(Supplementary Note 11)

A learning model generation method including a learning model generation step for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

(Supplementary Note 12)

The learning model generation method according to Supplementary Note 11, in which, in the learning model generation step, machine learning is further executed by using, as the training data, a content of a questionnaire of the sample person, and the learning model is generated with the inspection value and the content of the questionnaire as an explanatory variable and the parameter as an objective variable.

(Supplementary Note 13)

The learning model generation method according to Supplementary Note 12, in which the questionnaire includes a lifestyle of the sample person.

(Supplementary Note 14)

The learning model generation method according to Supplementary Note 11, in which the function is a function representing a transition of the inspection value after a set period elapses.

(Supplementary Note 15)

The learning model generation method according to Supplementary Note 11, further including a function setting step for setting the function based on transitions of inspection values of a plurality of persons for a specific period.

(Supplementary Note 16)

The learning model generation method according to Supplementary Note 11, in which a parameter of the function for the sample person is obtained from a transition of a past inspection value of the sample person over time.

(Supplementary Note 17)

An inspection value prediction method including:

an inspection value acquisition step for acquiring an inspection value of a person to be predicted;
a parameter prediction step for inputting the acquired inspection value to a learning model in which a relationship between an inspection value and a parameter of a function representing a transition of the inspection value is machine-learned and predicting the parameter of the person to be predicted; and
an inspection value prediction step for inputting the predicted parameter to the function and predicts a future inspection value of the person to be predicted.

(Supplementary Note 18)

The inspection value prediction method according to Supplementary Note 17, in which

the learning model performs machine learning on a relationship with a content of a questionnaire of a person in addition to an inspection value and a parameter of a function representing a transition of the inspection value,
in the inspection value acquisition step, a questionnaire of the person to be predicted is further acquired, and
in the parameter prediction step, the acquired inspection value and the content of the questionnaire are inputted to the learning model to predict the parameter of the person to be predicted.

(Supplementary Note 19)

The inspection value prediction method according to Supplementary Note 18, in which the questionnaire includes a lifestyle of the sample person.

(Supplementary Note 20)

The inspection value prediction method according to Supplementary Note 17, in which the function is a function representing a transition of the inspection value after a set period elapses.

(Supplementary Note 21)

A computer-readable recording medium having recorded therein a program including an instruction for causing a computer to execute a learning model generation step for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

(Supplementary Note 22)

The computer-readable recording medium according to Supplementary Note 21, in which, in the learning model generation step, machine learning is further executed by using, as the training data, a content of a questionnaire of the sample person, and the learning model is generated with the inspection value and the content of the questionnaire as an explanatory variable and the parameter as an objective variable.

(Supplementary Note 23)

The computer-readable recording medium according to Supplementary Note 22, in which the questionnaire includes a lifestyle of the sample person.

(Supplementary Note 24)

The computer-readable recording medium according to Supplementary Note 21, in which the function is a function representing a transition of the inspection value after a set period elapses.

(Supplementary Note 25)

The computer-readable recording medium according to Supplementary Note 21, in which the program causes the computer to further execute a function setting step for setting the function based on transitions of inspection values of a plurality of persons for a specific period.

(Supplementary Note 26)

The computer-readable recording medium according to Supplementary Note 21, in which a parameter of the function for the sample person is obtained from a transition of a past inspection value of the sample person over time.

(Supplementary Note 27)

A computer-readable recording medium having recorded therein a program including an instruction for causing a computer to execute:

an inspection value acquisition step for acquiring an inspection value of a person to be predicted;
a parameter prediction step for inputting the acquired inspection value to a learning model in which a relationship between an inspection value and a parameter of a function representing a transition of the inspection value is machine-learned and predicting the parameter of the person to be predicted; and
an inspection value prediction step for inputting the predicted parameter to the function and predicts a future inspection value of the person to be predicted.

(Supplementary Note 28)

The computer-readable recording medium according to Supplementary Note 27, in which

the learning model performs machine learning on a relationship with a content of a questionnaire of a person in addition to an inspection value and a parameter of a function representing a transition of the inspection value,
in the inspection value acquisition step, a questionnaire of the person to be predicted is further acquired, and
in the parameter prediction step, the acquired inspection value and the content of the questionnaire are inputted to the learning model to predict the parameter of the person to be predicted.

(Supplementary Note 29)

The computer-readable recording medium according to Supplementary Note 28, in which the questionnaire includes a lifestyle of the sample person.

(Supplementary Note 30)

The computer-readable recording medium according to Supplementary Note 27, in which the function is a function representing a transition of the inspection value after a set period elapses.
While the present invention has been particularly shown and described with reference to example embodiments thereof, the present invention is not limited to these example embodiments. It will be understood by those of ordinary skill in the art that various changes in form and details may be made therein without departing from the spirit and scope of the present invention as defined by the claims.
This application is based upon and claims the benefit of priority from Japanese patent application No.

2023-050360, filed on March 27, 2023, the disclosure of which is incorporated herein in its entirety by reference.

Industrial Applicability

As described above, according to the present disclosure, it is possible to improve interpretability by indicating a certain tendency in prediction of an inspection value of a user. The present disclosure is useful in the medical field.

Reference Signs List

| | |
|---|---|
| 10 | learning model generation apparatus (first example embodiment) |
| 11 | learning model generation unit |
| 12 | storage unit |
| 13 | model parameters |
| 20 | learning model generation apparatus (modification of first example embodiment) |
| 21 | function setting unit |
| 30 | database |
| 40 | inspection value prediction apparatus |
| 41 | inspection value acquisition unit |
| 42 | parameter prediction unit |
| 43 | inspection value prediction unit |
| 44 | learning model |
| 110 | computer |
| 111 | CPU |
| 112 | main memory |
| 113 | storage device |
| 114 | input interface |
| 115 | display controller |
| 116 | data reader/writer |
| 117 | communication interface |
| 118 | input device |
| 119 | display device |
| 120 | recording medium |
| 121 | bus |

**Claims**

1. A learning model generation apparatus comprising a learning model generation means for, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

2. The learning model generation apparatus according to claim 1, wherein the learning model generation means further executes machine learning by using, as the training data, a content of a questionnaire of the sample person, and generates the learning model with the inspection value and the content of the questionnaire as an explanatory variable and the parameter as an objective variable.

3. The learning model generation apparatus according to claim 2, wherein the questionnaire includes a lifestyle of the sample person.

4. The learning model generation apparatus according to claim 1, wherein the function is a function representing a transition of the inspection value after a set period elapses.

5. The learning model generation apparatus according to claim 1, further comprising a function setting means for setting the function based on transitions of inspection values of a plurality of persons for a specific period.

6. The learning model generation apparatus according to claim 1, wherein a parameter of the function for the sample person is obtained from a transition of a past inspection value of the sample person over time.

7. An inspection value prediction apparatus comprising:

   an inspection value acquisition means for acquiring an inspection value of a person to be predicted;
   a parameter prediction means for inputting the acquired inspection value to a learning model in which a relationship between an inspection value and a parameter of a function representing a transition of the inspection value is machine-learned and predicting the parameter of the person to be predicted; and
   an inspection value prediction means for inputting the predicted parameter to the function and predicting a future inspection value of the person to be predicted.

8. The inspection value prediction apparatus according to claim 7, wherein

   the learning model performs machine learning on a relationship with a content of a questionnaire of a person in addition to an inspection value and a parameter of a function representing a transition of the inspection value,
   the inspection value acquisition means further acquires a questionnaire of the person to be predicted, and
   the parameter prediction means inputs the acquired inspection value and the content of the questionnaire to the learning model to predict the parameter of the person to be predicted.

9. The inspection value prediction apparatus according to claim 8, wherein the questionnaire includes a lifestyle of a person.

10. The inspection value prediction apparatus according to claim 7, wherein the function is a function representing a transition of the inspection value after a set period elapses.

11. A learning model generation method comprising, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

12. An inspection value prediction method comprising:

acquiring an inspection value of a person to be predicted;
inputting the acquired inspection value to a learning model in which a relationship between an inspection value and a parameter of a function representing a transition of the inspection value is machine-learned and predicting the parameter of the person to be predicted; and
inputting the predicted parameter to the function and predicting a future inspection value of the person to be predicted.

13. A computer-readable recording medium having recorded therein a program including an instruction for causing a computer to execute, in a case where a function representing a transition of an inspection value obtained from inspection on a person is set, executing machine learning with, as training data, an inspection value of a sample person and a parameter of the function for the sample person and generating a learning model with the inspection value as an explanatory variable and the parameter as an objective variable.

14. A computer-readable recording medium having recorded therein a program including an instruction for causing a computer to execute:

acquiring an inspection value of a person to be predicted;
inputting the acquired inspection value to a learning model in which a relationship between an inspection value and a parameter of a function representing a transition of the inspection value is machine-learned and predicting the parameter of the person to be predicted; and inputting the predicted parameter to the function and predicting a future inspection value of the person to be predicted.

Fig.1

10

LEARNING MODEL GENERATION
APPARATUS

11

LEARNING MODEL
GENERATION UNIT

Fig.2

LEARNING MODEL GENERATION
APPARATUS — 10

DATABASE — 30

LEARNING MODEL
GENERATION UNIT — 11

STORAGE UNIT — 12

MODEL PARAMETER — 13

Fig.3

INSPECTION VALUE

ELAPSED YEARS

——— APPROXIMATE CURVE (alogt+b)

——— ACTUAL VALUE

Fig.4

TRAINING DATA

| ID NUMBER | PARAMETER a | AFTER 1 YEAR | | | AFTER 2 YEAR | | | AFTER 1 YEAR | | |
|---|---|---|---|---|---|---|---|---|---|---|
| | | IV | QUESTIONNAIRE | | IV | QUESTIONNAIRE | | IV | QUESTIONNAIRE | |
| | | | SMOKING | DRINKING FREQUENCY | | SMOKING | DRINKING FREQUENCY | | SMOKING | DRINKING FREQUENCY |
| 1 | 1.25 | 108 | Yes | NOT DRINK | 102 | Yes | NOT DRINK | 110 | Yes | ONCE A MONTH |
| 2 | 1.37 | 108 | No | THREE TIMES A WEEK | 115 | No | THREE TIMES A WEEK | 130 | Yes | EVERY DAY |
| 3 | 1.17 | 108 | No | ONCE A MONTH | 106 | No | ONCE A WEEK | 114 | No | THREE TIMES A WEEK |
| 4 | 1.24 | 108 | Yes | ONCE A WEEK | 118 | Yes | ONCE A WEEK | 127 | Yes | ONCE A WEEK |
| ... | | | | | | | | | | |

*IV: INSPECTION VALUE

Fig.5

Flowchart:

Start
↓
ACQUIRE SET OF TRAINING DATA — A1
↓
PERFORM MACHINE LEARNING TO UPDATE PARAMETERS OF LEARNING MODEL — A2
↓
HAS MACHINE LEARNING BEEN ENDED FOR ALL TRAINING DATA? — A3
No → (back to A1)
Yes ↓
STORE PARAMETERS OF LATEST LEARNING MODEL — A4
↓
End

Fig.6

LEARNING MODEL GENERATION
APPARATUS                          20

30

DATABASE ──────── FUNCTION SETTING UNIT          21

                  LEARNING MODEL
                  GENERATION UNIT               11

                  STORAGE UNIT                  12

                      MODEL PARAMETER           13

Fig.7

40

INSPECTION VALUE PREDICTION
APPARATUS

41

INSPECTION VALUE
ACQUISITION UNIT

42

PARAMETER PREDICTION
UNIT

43

INSPECTION VALUE
PREDICTION UNIT

Fig.8

40

INSPECTION VALUE PREDICTION APPARATUS

41

INSPECTION VALUE
QUESTIONNAIRE →

INSPECTION VALUE
ACQUISITION UNIT

42

PARAMETER PREDICTION
UNIT

44

LEARNING
MODEL

43

INSPECTION VALUE
PREDICTION UNIT

PREDICTION
VALUE

13

MODEL PARAMETER

Fig.9

```
            ┌─────────────┐
            │    Start    │
            └──────┬──────┘
                   │
                   ▼
    ┌──────────────────────────────┐
    │ ACQUIRE INSPECTION  VALUE AND │── B1
    │         QUESTIONNAIRE         │
    └───────────────┬──────────────┘
                    │
                    ▼
    ┌──────────────────────────────┐
    │ PREDICT FUNCTION PARAMETER    │── B2
    │ USING LEARNING MODEL          │
    └───────────────┬──────────────┘
                    │
                    ▼
    ┌──────────────────────────────┐
    │ PREDICT FUTURE INSPECTION     │── B3
    │ VALUE USING FUNCTION          │
    └───────────────┬──────────────┘
                    │
                    ▼
    ┌──────────────────────────────┐
    │ PRESENT PREDICTED INSPECTION  │── B4
    └───────────────┬──────────────┘
                    │
                    ▼
            ┌─────────────┐
            │     End     │
            └─────────────┘
```

Fig.10

110

COMPUTER

| 111 | 112 | 113 |
| CPU | MAIN MEMORY | STORAGE DEVICE |

121

| 114 | 115 | 116 | 117 |
| INPUT INTERFACE | DISPLAY CONTROLLER | DATA READER/ WRITER | COMMUNICATION INTERFACE |

| INPUT DEVICE | DISPLAY DEVICE | RECORDING MEDIUM |

118    119    120    OTHER COMPUTER etc.

## INTERNATIONAL SEARCH REPORT

International application No.

**PCT/JP2024/009956**

### A. CLASSIFICATION OF SUBJECT MATTER

*G16H 50/00*(2018.01)i; *G06N 20/00*(2019.01)i
FI:   G16H50/00; G06N20/00 130

According to International Patent Classification (IPC) or to both national classification and IPC

### B. FIELDS SEARCHED

Minimum documentation searched (classification system followed by classification symbols)

G16H10/00-80/00; G06N20/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2019-128904 A (NEC SOLUTION INNOVATORS LTD.) 01 August 2019 (2019-08-01)<br>paragraphs [0027], [0032], [0064], [0074], [0076] | 1-14 |
| A | WO 2019/187933 A1 (NEC SOLUTION INNOVATORS LTD.) 03 October 2019<br>(2019-10-03)<br>paragraphs [0020], [0021] | 1-14 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **12 April 2024** | **07 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/009956**

| Patent document cited in search report | | | Publication date (day/month/year) | Patent family member(s) | Publication date (day/month/year) |
|---|---|---|---|---|---|
| JP | 2019-128904 | A | 01 August 2019 | (Family: none) | |
| WO | 2019/187933 | A1 | 03 October 2019 | US 2021/0257072 A1 paragraphs [0036], [0037] EP 3780001 A1 CN 111971756 A | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023050360 A

**Non-patent literature cited in the description**

- **ATSUSHI TANIGUCHI, HIROSHI TAMANO**. Learning Medical Checkup Prediction Model with Interpretable Interannual Prediction. *FIT2018 (17th Information Science and Technology Forum)*, 2018, 45-50